# EUROPÄISCHE PATENTSCHRIFT

(11) **EP 1 123 278 B1**
(45) Veröffentlichungstag und Bekanntmachung des Hinweises auf die Patenterteilung: **21.06.2006**
(21) Anmeldenummer: 99953799.6
(22) Anmeldetag: 13.10.1999
(51) Int. Cl.: C07D 209/52, C07B 37/10

(54) **VERFAHREN ZUR HERSTELLUNG VON (+)-EXO-6-PHENYL-3-AZABICYCLO- 3.2.0|HEPTANEN**
METHOD FOR PRODUCING (+)-EXO-6-PHENYL-3-AZABICYCLO- 3.2.0|HEPTANES
PROCEDE DE PRODUCTION DE (+)-EXO-6-PHENYL-3-AZABICYCLO- 3.2.0|HEPTANES

(30) Priorität: 21.10.1998 DE 19848521
(43) Veröffentlichungstag der Anmeldung: 16.08.2001
(73) Patentinhaber: Abbott GmbH & Co. KG, 65205 Wiesbaden (DE)
(72) Erfinder: BISCHOF, Norbert, D-68163 Mannheim (DE); BUSCHMANN, Ernst, D-67069 Ludwigshafen (DE); HENNINGSEN, Michael, D-67227 Frankenthal (DE); KREI, Georg, Arnold, D-67122 Altrip (DE); MUNSCHAUER, Rainer, 67435 Neustadt (DE); PFEIFFER, Thomas, D-67459 Böhl-Iggelheim (DE); STEINER, Gerd, D-67281 Kirchheim (DE); VIERGUTZ, Wolfgang, D-67067 Ludwigshafen (DE)
(74) Vertreter: Grünecker, Kinkeldey, Stockmair & Schwanhäusser Anwaltssozietät
(86) Internationale Anmeldenummer: PCT/EP1999/007682
(87) Internationale Veröffentlichungsnummer: WO 2000/023423

(56) Entgegenhaltungen:
- DE-A- 4 243 287
- DE-A- 4 427 648
- STEINER, GERD ET AL: "Diastereoselective synthesis of exo-6-aryl-3-azabicyclo[3.2.0]heptane derivatives by intramolecular [2+2] photocycloadditions of diallylic amines" HETEROCYCLES., Bd. 40, Nr. 1, 1995, Seiten 319-330, XP002129479 ELSEVIER SCIENCE PUBLISHERS B.V. AMSTERDAM., NL ISSN: 0385-5414 in der Anmeldung erwähnt

## Beschreibung

Die Erfindung betrifft ein einfaches und technisch gut durchführbares Verfahren zur Herstellung von (+)-exo-6-Phenyl-3-aza-bicyclo-[3.2.0]heptanen. Diese Verbindungen haben als Vorprodukte für Neuroleptika Bedeutung erlangt (DE 4219973, 4427647, 4243287 4427648).

6-Phenyl-3-azabicyclo-[3.2.0]heptane sind durch photolytische Zyklisierung von Bisallylammoniumsalzen 1 zugänglich. Bei der Photozyklisierung entstehen vier Stereoisomere 2 (Schema 1).

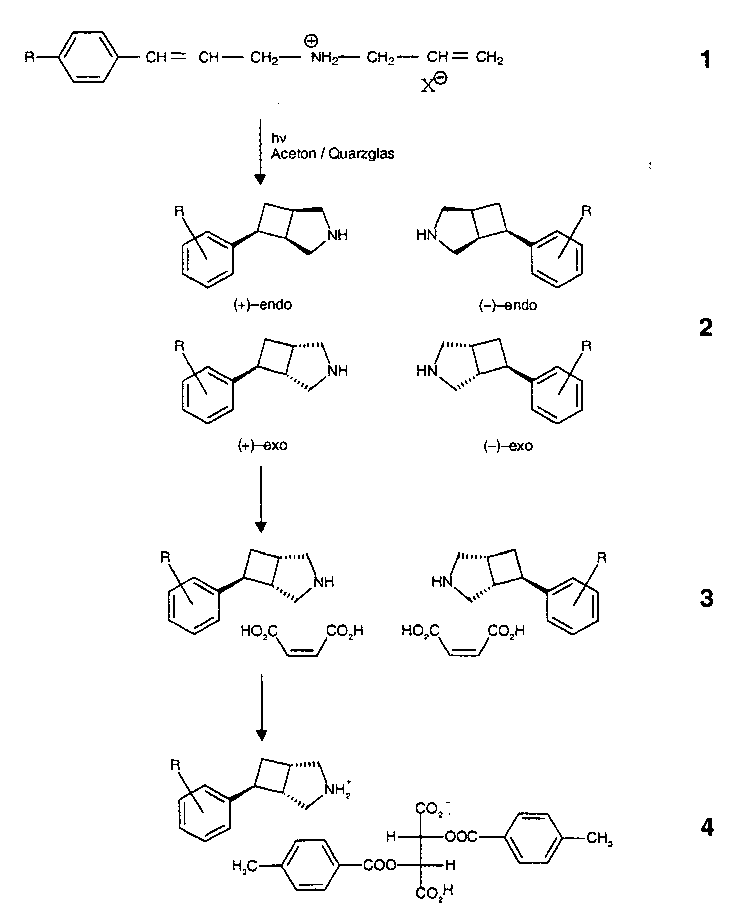

Die unerwünschten endo-Isomere werden durch eine Maleinatfällung zu 3 abgetrennt, anschließend wird das (+)-exo-Isomere als Ditoluoyltartrat 4 isoliert (Heterocycles 40, 319 (1995)). Das Verfahren ist im Labormaßstab sehr gut zur Herstellung reiner 6-Phenyl-3-azabicyclo[3.2.0]heptane durchführbar. Bei der technischen Herstellung gibt es jedoch schwerwiegende Probleme:

Die Auftrennung des Isomerengemisches 2 erfordert eine Vielzahl von Verfahrensschritten:
Neutralisation der Rohlösung der Photozyklisierung;
Extraktion des Isomerengemischs mit Ether;
Trocknen der Lösung über Na₂SO₄;
Abfiltrieren des Na₂SO₄;
Eindampfen der Lösung;
Lösen des Rückstands in Isopropanol;
Fällung des Maleinats mit Maleinsäure;
Abfiltrieren des Maleinats;
Suspendieren des Maleinats in Wasser und Zugabe von Alkali;
Extraktion des exo-Amins mit Ether;
Eindampfen des Lösungsmittels;
Lösen der exo-Isomere in Ethanol;
Fällung des Ditoluoyltartrats mit (-)-Ditoluoylweinsäure; und Umkristallisation des Ditoluoyltartrats aus Propanol/Wasser.

Bei dieser Synthese entstehen große Mengen an Reststoffen, die entsorgt werden müssen. Weiter muß die Photozyklisierung in einer Quarzapparatur vorgenommen werden. Insbesondere große Quarzapparate sind jedoch schwer herzustellen, leicht zerbrechlich und nur mit hohem technischen Aufwand zu reparieren.

Bei der Übertragung der Labormethode in den technischen Maßstab kommt es auf dem Quarz-Lampenkörper darüber hinaus zur Bildung störender Beläge. Die Umsetzung wird dadurch verlangsamt und ist in manchen Fällen unvollständig.

Zur Fällung des Ditoluoyltartrates 4, ein Salz bestehend aus jeweils einem Äquivalent (-)-Ditoluoylweinsäure (= DTW) und 6-Phenyl-3-azabicycloheptan, wird die teure (-)-Ditoluoylweinsäure im Überschuß von 130 % eingesetzt. Dadurch werden auch hier große Mengen an teurem Reststoff erzeugt.

Bei der abschließenden Umkristallisation von 4 aus Propanol/Wasser kommt es bei den längeren Aufheiz- und Abkühlzeiten im technischen Betrieb großer Rührkessel zur partiellen Zersetzung des Ditoluoyltartrates 4, so daß die im Labor gefundenen Ausbeuten nicht erreicht werden.

Es wurde nun ein Verfahren gefunden, das die Probleme, die bei der Übertragung der Laborsynthese in den technischen Maßstab entstehen, löst.

Gegenstand der Erfindung ist ein Verfahren zur Herstellung von (+)-exo-6-Phenyl-3-azabicyclo-[3.2.0]heptanen der Formel I 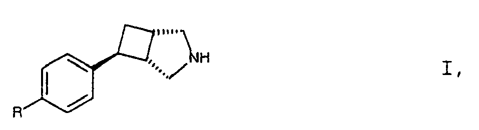 in der R für Wasserstoff, Chlor, Brom, Fluor oder Methoxy steht, dadurch gekennzeichnet, daß man ein Bisallylammoniumsalz der Formel II 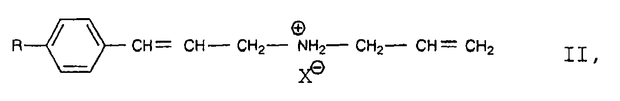 in der R die oben genannten Bedeutungen hat und X- für ein Anion steht in einem inerten Lösungsmittel unter Zusatz eines Sensibilisators in einer Glasapparatur photozyklisiert und mit (-)-Ditoluoylweinsäure aus dem erhaltenen Gemisch das gewünschte (+)-exo-Isomer I als Ditoluoyltartrat ausfällt und dieses gegebenenfalls aus einer Alkohol/Wasser-Mischung umkristallisiert,
mit der Maßgabe, dass die Glasapparatur keine Quarzapparatur ist.

X- bedeutet ein Halogenoid, Sulfat, Phosphat, Nitrat oder ein anderes, geeignetes Anion, bevorzugt sind Chlorid und Phosphat.

Die Umsetzung erfolgt in einem inerten Lösungsmittel wie Wasser, einem Alkohol wie Methanol, Ethanol, Propanol, einem Keton wie Aceton, Methylethylketon, einem Amid wie Dimethylformamid oder N-Methylpyrrolidon, bevorzugt ist Aceton.

Zweckmäßigerweise wird die Photozyklisierung unter Zusatz eines Sensibilisators, wie Benzophenon, Acetophenon, am Aromaten substituierte Benzophenone oder Acetophenone (vorzugsweise Acetophenon), bei Temperaturen von 0 bis 80°C, insbesondere bei 15 bis 30°C durchgeführt. Als Lichtquellen eignen sich Quecksilberhochdrucklampen oder vergleichbare Lichtquellen.

Ebenso wie bei der literaturbekannten Zyklisierung in Quarzapparaturen werden Isomerengemische erhalten, in denen die Verbindungen I zu etwa 45 % vorliegen. Der Rest besteht zu etwa 45 % aus dem (-)-exo-Isomeren und von den (+)- und (-)-endo-Isomeren werden jeweils etwa 5 % gebildet.

Das nach dem neuen Verfahren erhaltene (+)-exo-6-Phenyl-3-aza-bicyclo[3.2.0]heptan läßt sich mit (-)-Ditoluoylweinsäure (= DTW) direkt aus dem Rohgemisch der Photozyklisierung ausfällen. Das (-)-exo-Enantiomer und die endo-Stereoisomere bleiben dabei überraschenderweise in Lösung. Das neue Verfahren zur Abtrennung der gewünschten (+)-exo-Isomere besteht damit nur noch aus wenigen Verfahrensschritten. Alle zeitaufwendigen Extraktionsschritte entfallen.

Über die Photozyklisierung von Bisallylaminen ist wenig bekannt (Heterocycles 40, 319 (1995) und die dort zitierte Literatur). Überraschenderweise ergab sich, daß die Photozyklisierung auch in einfachen Glasapparaten durchgeführt werden kann, wenn man einen geeigneten Sensibilisator, z.B. vorzugsweise einen Triplett-Sensibilisator, zusetzt. Ein besonders guter Sensibilisator ist Acetophenon. In den Glasapparaten kommt es dann nicht zur störenden Belagbildung auf dem Lampenkörper.

Weiter zeigt sich, daß sich die DTW-Menge von 130 % auf 20 bis 30 Mol.-%, bezogen auf eingesetztes Rohbicycloheptan, reduzieren läßt. Statt der Salze, die aus einem Äquivalent Bicycloheptan und einem Äquivalent DTW zusammengesetzt sind, werden Salze ausgefällt, die aus einem Äquivalent DTW und zwei Äquivalenten Bicycloheptan bestehen. Die Ausbeuten und Reinheiten am gewünschten Stereoisomer I bleiben dabei unverändert.

Nach Abschluß der Photozyklisierung wird, falls das Lösungsmittel für die Photozyklisierung kein Alkohol war, ein Lösungsmitteltausch vorgenommen, das heißt, das eingesetzte Lösungsmittel wird eingedampft und durch einen geeigneten Alkohol wie z.B. Methanol, Ethanol, n-Propanol, Isopropanol, n-Butanol, sec.-Butanol, Isobutanol, n-Pentanol oder durch ein Alkoholgemisch ersetzt. Bevorzugtes Lösungsmittel ist Ethanol. Dann wird Wasser zugegeben und mit einer geeigneten Base einen pH von 6,5 bis 7,5, bevorzugt 7, eingestellt. Geeignete Basen hierfür sind NaOH, KOH, Na₂CO₃, K₂CO₃ oder Amine wie Ammoniak, Diethylamin oder Triethylamin. Bevorzugt ist Triethylamin.

Danach wird aus der Rohlösung mit einer alkoholischen (bevorzugt ethanolischen) Lösung von (-)-Ditoluoylweinsäure das gewünschte (+)-exo-Enantiomer der Formel III als Ditoluoyltartrat 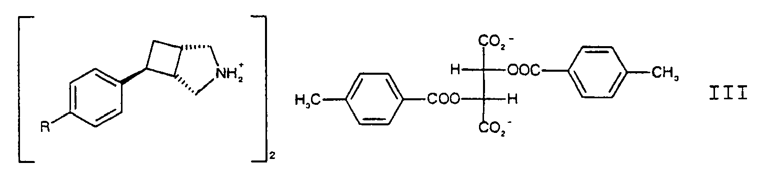 gefällt, wobei jeweils ein Äquivalent DTW mit zwei Äquivalenten des bicyclischen Amins auskristallisieren. Die besten Ausbeuten und Produktreinheiten werden erhalten, wenn man zwischen 20 und 35 Mol.-% (-)-DTW, bezogen auf eingesetztes Isomerengemisch von I, verwendet. Bevorzugt sind 25 - 30 Mol.-%. Man erhält die bicyclischen Amine als Ditoluoyltartrate III in einer chemischen Reinheit von > 99 % und optischen Reinheiten von > 96 % als (+)-exo-Isomere.

Zur weiteren Verbesserung der optischen Reinheit können diese Salze umkristallisiert werden.

Als Lösungsmittel für die Umkristallisation eignen sich am besten Alkohol/Wasser-Mischungen. Bevorzugt wird Propanol/Wasser verwendet. Durch die Umkristallisation werden ausgezeichnete chemische und optische Reinheiten (jeweils > 99,5 %) erzielt.

Setzt man bei der Umkristallisation eine geringe Menge (ca. 1 bis 5 % der Gesamtmenge) an einem tertiären Amin, vorzugsweise Triethylamin, zu, so läßt sich die Zersetzung des Ditoluoyltartrats III bei den längeren Aufheiz- und Abkühlzeiten in den größeren Technikumsapparaten vermeiden.

Das folgende Beispiel erläutert die Erfindung.

### Beispiel 1

### a) Photozyklisierung

335,7 kg N-Allyl-N[3-(4-fluorphenyl)allyl]-amin x H₃PO₄ (IIa) wurden in 225,6 kg Acetophenon und 4300 1 Aceton suspendiert. Nach Zugabe von 71,7 kg Phosphorsäure (85 %ig) und 609,6 1 Wasser bildete sich eine homogene Lösung, die nach 5tägiger Bestrahlung mit einer 40 kW-Quecksilberhochdrucklampe vollständig umgesetzt war. Es hatte sich ein Stereoisomerengemisch von 6-(4-Fluorphenyl)-3-azabicyclo[3.2.0]-heptan gebildet. Das Verhältnis exo- : endo-Isomer betrug 90, 6:9,4 bis 95,8 : 4,2.

### b) Racematspaltung mit (-)-Ditoluoylweinsäure

Das gemäß a) erhaltene Produkt der Photozyklisierung wurden zum Rückfluß erwärmt. Danach wurde Aceton abdestilliert, bis eine Innentemperatur von 60°C erreicht war. Es wurden 680 1 Ethanol und 470 1 Wasser zugegeben und mit Triethylamin auf pH 7 eingestellt. Bei 40°C wurden 490,8 kg einer 25 %igen, ethanolischen Lösung von (-)-Ditoluoylweinsäure zugesetzt. Die Lösung wurde auf -5°C abgekühlt und der Feststoff (IIIa) abzentrifugiert. Der in der Zentrifuge verbleibende Feststoff wurde mit einer Mischung aus 250 1 Wasser und 250 1 Ethanol gewaschen. Das Produkt wurde im Vakuumtrockenschrank bei 50°C getrocknet. Ausbeute: 145 kg Ditoluoyltartrat (65 %), optische Reinheit: 97,3 %, chemische Reinheit: 99,3 %.

### c) Umkristallisation des Tartrates

225 kg des Ditoluoyltartrats (IIIa) wurden in 1 020 1 n-Propanol, 1020 1 Wasser und 144 1 Triethylamin zum Rückfluß erwärmt. Man kühlte auf -5°C ab und trennte das Produkt über eine Zentrifuge ab. Nach Trocknung im Vakuumtrockenschrank bei 50°C wurden 175,8 kg (+)-exo-6-Phenyl-3-aza-bicyclo-[3.2.0]heptanen (78,4 %) erhalten. Chemische Reinheit: 99,8 %, optische Reinheit: 99,8 %.

## Patentansprüche

1. Verfahren zur Herstellung von (+)-exo-6-Phenyl-3-azabicyclo[3.2.0]heptanen der Formel I 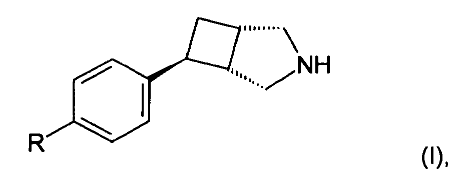 in der R für Wasserstoff, Chlor, Brom, Fluor oder Methoxy steht, **dadurch gekennzeichnet, dass** man ein Bisallylammoniumsalz der Formel II 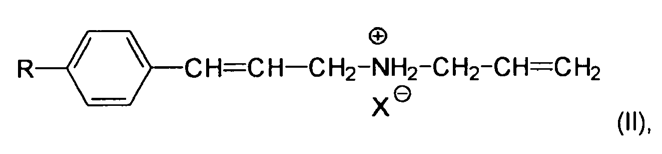 in der R die oben genannten Bedeutungen hat und X⁻ für ein Anion steht, in einem inerten Lösungsmittel unter Zusatz eines Sensibilisators in einer Glasapparatur photozyklisiert und mit (-)-Ditoluoylweinsäure aus dem erhaltenen Gemisch das gewünschte (+)-exo-Isomer I als Ditoluoyltartrat ausfällt und dieses gegebenenfalls aus einer Alkohol/Wasser-Mischung umkristallisiert,
mit der Maßgabe, dass die Glasapparatur keine Quarzglasapparatur ist.

2. Verfahren nach Anspruch 1, **dadurch gekennzeichnet, dass** man als Sensibilisator Acetophenon verwendet.

3. Verfahren nach Anspruch 1, **dadurch gekennzeichnet, dass** man für die Fällung und Umkristallisation Ethanol oder n-Propanol verwendet.

4. Verfahren nach Anspruch 1, **dadurch gekennzeichnet, dass** man bei der Umkristallisation des Ditoluoyltartrats ein tertiäres Amin zusetzt.

## Claims

1. Method for producing (+)-exo-6-phenyl-3-azabicyclo[3.2.0]heptanes of the formula I 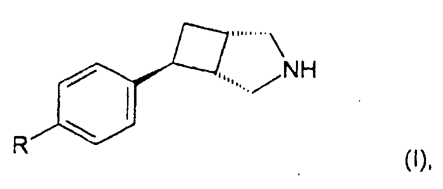 in which R represents hydrogen, chlorine, bromine, fluorine or methoxy, **characterized in that** a bisallylammonium salt of the formula II 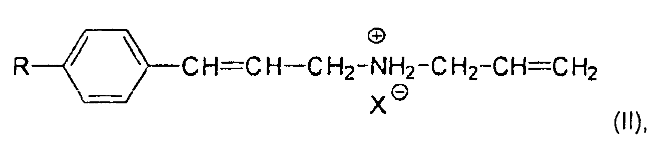 in which R has the abovementioned meanings and X⁻ represents an anion, is subjected to photocyclization in an inert solvent in a glass apparatus, with the addition of a sensitizer, and the desired (+)-exo isomer I is precipitated out of the resulting mixture, using (-)-ditoluoyltartaric acid, as the ditoluoyltartrate and this is optionally recrystallized from an alcohol/water mixture,
with the proviso that the glass apparatus is not a quartz glass apparatus.

2. Method according to claim 1, **characterized in that** acetophenone is used as the sensitizer.

3. Method according to claim 1, **characterized in that** ethanol or n-propanol is used for the precipitation and recrystallization.

4. Method according to claim 1, **characterized in that** a tertiary amine is added in the recrystallization of the ditoluoyltartrate.

## Revendications

1. Procédé de préparation de (+)-exo-6-phényl-3-azabicyclo[3.2.0]-heptanes de formule I 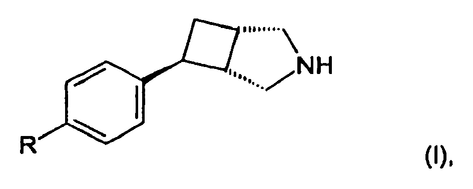 dans laquelle R représente l'hydrogène, le chlore, le brome, le fluor ou un méthoxy, **caractérisé en ce que** l'on soumet un sel de bisallylammonium de formule II 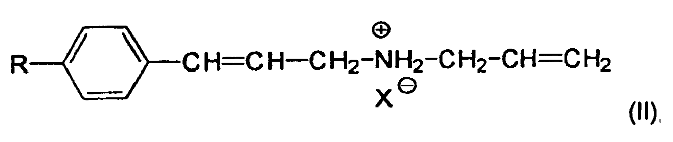 dans laquelle R a les significations indiquées ci-dessus et X⁻ représente un anion, à une photocyclisation dans un solvant inerte en présence d'un agent sensibilisant dans un appareil en verre, et, à partir du mélange obtenu, on précipite avec de l'acide (-)-ditoluoyltartrique l'isomère (+)-exo I désiré sous forme du ditoluoyltartrate, que l'on recristallise éventuellement dans un mélange alcool/eau,
à condition que l'appareil en verre ne soit pas un appareil en verre de quartz.

2. Procédé selon la revendication 1, **caractérisé en ce que** l'on utilise de l'acétophénone comme agent sensibilisant.

3. Procédé selon la revendication 1, **caractérisé en ce que** l'on utilise de l'éthanol ou du n-propanol pour la précipitation et la recristallisation.

4. Procédé selon la revendication 1, **caractérisé en ce que** l'on ajoute une amine tertiaire lors de la recristallisation du ditoluoyltartrate.
